Europäisches Patentamt

European Patent Office

Office européen des brevets

(11) Publication number: **0 198 514 B1**

# EUROPEAN PATENT SPECIFICATION

(45) Date of publication of patent specification: **21.04.93** (51) Int. Cl.⁵: **B29C 41/14, A61B 19/04**

(21) Application number: **86105454.2**

(22) Date of filing: **02.09.83**

(60) Publication number of the earlier application in accordance with Art.76 EPC: **0 105 613**

(54) **Dipped rubber article.**

(30) Priority: **03.09.82 GB 8225200**
**30.11.82 US 445436**

(43) Date of publication of application:
**22.10.86 Bulletin 86/43**

(45) Publication of the grant of the patent:
**21.04.93 Bulletin 93/16**

(84) Designated Contracting States:
**DE FR GB**

(56) References cited:
**WO-A-81/00345**
**FR-A- 1 434 453**
**FR-A- 2 193 710**
**US-A- 3 813 695**
**US-A- 4 143 109**

(73) Proprietor: **LRC PRODUCTS LIMITED**
**North Circular Road**
**London, E4 8OA(GB)**

(72) Inventor: **James, Michael Howard**
**Oakleigh House 12 West Common Way**
**Harpenden Hertfordshire(GB)**
Inventor: **Bratby, David Michael**
**18 Deborah Court Victoria Road**
**South Woodford London E.18(GB)**
Inventor: **Blackley, David Charles**
**100 Berkeley Avenue Chesham**
**Buckinghamshire HP5 2RS(GB)**
Inventor: **Duck, Roger**
**29 Woodstock Road**
**Upper Walthamstow London E17(GB)**
Inventor: **Podell, Howard Irwin**
**28 Beachfront Lane**
**New Rochelle New York 10805(US)**
Inventor: **Goldstein, Albert**
**87 Glenwood Drive**
**Tinton Falls New Jersey 07724(US)**

(74) Representative: **Spencer, Graham Easdale et al**
**A.A. Thornton & CO Northumberland House**
**303-306, High Holborn**
**London WC1V 7LE (GB)**

Rank Xerox (UK) Business Services
(3.10/3.5x/3.0.1)

**Description**

The present invention is concerned with a process for making flexible dipped rubber articles and, in particular, thin-walled rubber gloves of the kind used by surgeons.

Surgeon's gloves are difficult to don and to facilitate donning a powdered lubricant, such as particulate epichlorhydrin-treated maize starch, is conventionally applied to the inner surface of the gloves. There is a risk of such powdered lubricant escaping from the interior of the glove to contaminate the surgical field, the lubricant escaping either during donning or, as sometimes happens, if the glove is punctured during an operation.

Proposals have been made for polymeric lubricant coatings which are bonded to the inner surface of such gloves and which, because they are bonded, cannot escape from the glove.

Examples of such proposals are in U.S. Patents 4070713 and 4143109, which disclose gloves which have an inner layer of elastomeric material with particulate lubricant embedded therein, and U.S. Patents 3813695, 3856561 and 4302852, which disclose surgeon's gloves with various polymeric slip coatings bonded to the inner surface thereof. French Patent 1434453 is a similar disclosure relating to dipped rubber articles in general.

U.S. Patent 3813695 ("the Podell patent") describes a surgeon's glove in which the glove material is formed of a laminate consisting of an outer layer of flexible material, for example rubber, and an inner layer of hydrophilic plastic material (such as a hydrogel polymer), the inner and outer layers being bonded together.

There are many known hydrogel polymers, examples of which are polyvinyl pyrrolidone, polyhydroxyethyl acrylate or methacrylate, polyhydroxypropyl acrylate or methacrylate, and copolymers of these with each other or with acrylic or methacrylic acid, acrylic or methacrylic esters or vinyl pyridine.

There are many disclosures of the coating of rubber articles, such as catheters and bathing caps, with such hydrogel polymers by dipping in a solution of a hydrophilic, hydrogel-forming polymer and curing the resulting polymer layer.

Examples of such disclosures include U.S. Patents 3326742, 3585103, 3607473, 3745042, 3901755, 3925138, 3930076, 3940533, 3966530, 4024317, 4110495, and 4125477, and British Patents 1028446 and 859297.

It is conventional to make such flexible rubber articles by dipping a suitably shaped former in a rubber latex; the rubber article is vulcanised on the former before being stripped from the latter. We have now found that in the production of hydrogel-coated flexible rubber articles, a substantial advantage is obtained if the hydrogel layer is applied to the rubber before the latter is vulcanised, the hydrogel polymer being cured and the rubber vulcanised simultaneously in a single heat treatment. The surprising advantage obtained by this procedure is that the skin-contacting (hydrogel) surface has a much cooler feel; this may be because there is greater vapour transmission through the final coated article.

According to the present invention, therefore, there is provided a process for making a flexible dipped rubber article having a lubricating layer formed from a hydrogel polymer bonded thereto so as to provide a skin-contacting surface of said article, characterised in that the lubricating layer is formed by applying a coating of a solution of a hydrophilic hydrogel-forming polymer to the dipped rubber article prior to the complete vulcanisation thereof, and heating the coated rubber article to cure the polymer and to complete vulcanisation of the rubber.

The polymer solution is preferably applied by dipping.

Any suitable hydrogel polymer may be used in the present invention, including the hydrogel polymers described in the prior documents mentioned above. It is preferred to use the hydrogel polymers which are copolymers of 2-hydroxyethyl methacrylate (HEMA) with methacrylic acid (MAA) and/or with 2-ethylhexyl acrylate (EHA) which are described in our Application 105613 (83305099.0).

The curing or cross-linking of the hydrogel polymer generally reduces the water-absorption of the polymer. After cross-linking, the layer may be surface treated with a physiologically acceptable surfactant or long chain fatty amine; this can enhance the lubricity of the layer with respect to damp skin. Such surfactants and fatty amines improve the lubricity with respect to damp skin for a wide range of hydrogel polymers as well as the specific 2-hydroxyethyl methacrylate copolymers referred to above. The use of such surfactants and fatty amines is fully described in our Application 105613 (83305099.0) already referred to.

An article made according to the invention is preferably treated with a silicone liquid so as to reduce the surface tack on any surfaces not coated with a layer formed from the hydrogel polymer; this treatment is preferably carried out at the same time as treatment with a surfactant as mentioned above. It is preferred that treatment with a silicone (such as medical grade polydimethyl siloxane) is carried out with a bath

2

containing at least 0.05% by weight of silicone (for example, 0.05 to 0.4% by weight).

The rubber used in the article according to the present invention may be a natural or synthetic rubber; natural rubber is preferred.

The process according to the invention preferably comprises the steps of:

(a) forming a rubber article by dipping a suitably shaped former in a rubber latex,

(b) leaching the rubber article on the former in hot water,

(c) priming the rubber surface of the article on the former by means of a dilute acid,

(d) rinsing the primed surface in water or aqueous alkali,

(e) dipping said article, while still on the former, in a solution of hydrophilic, hydrogel-forming polymer and a curing agent therefor,

(f) heating the resulting coating so that the resulting hydrogel polymer is bonded to the rubber and so that the rubber is vulcanised and the polymer is simultaneously cured, and

(g) stripping the resulting article from the former.

This process may optionally comprise the additional steps of:

(h) applying a solution of surfactant material containing silicone to the article (for example, by tumbling in such a solution), optionally after washing, and

(i) heating the resulting coating of surfactant material so as to fix the slip properties of the coating.

Our tests have shown that, following step (i), the damp skin slip properties and the dry skin slip properties of the coating formed from the hydrogel polymer are advantageously not impaired by subsequent washing (that is, surfactant material is not leached out to any substantial extent on washing of the coated article).

The application of the solution of surfactant material provides a substantially tack-free outer surface (that is, the surface not coated with hydrogel polymer), in addition to the inner surface, which is, of course, advantageous.

The rubber surface to which the hydrogel polymer is bonded may also be primed by dipping in, for example, a solution of an aluminium salt after priming with dilute acid.

It is a feature of the present invention that the production of the dipped rubber article, leaching, priming, application of hydrogel polymer layer, and vulcanisation of the rubber and curing of the polymer can all be carried out in a continuous operation.

The present invention has been described primarily, with reference to surgeons' gloves; it is, however, applicable to other skin- or tissue-contacting flexible rubber articles, such as condoms, gloves used by doctors and veterinary surgeons for examination purposes (such gloves being often donned with dry hands), catheters, urethers, sheets and sheath-type incontinence devices.

When the present invention is used for articles such as urethers and catheters, the layer formed from hydrogel polymer is provided on the outer surface (this being the skin-contacting surface); for condoms the layer formed from hydrogel polymer may be provided on the inner surface and/or on the outer surface.

In order that the present invention may be more fully understood, the following Examples and Comparative Examples are given by way of illustration only.

Example 1

A thin dipped surgeons glove of natural rubber latex was leached with sulphuric acid, rinsed, primed by dipping in aluminium sulphate solution, dried out completely and then dipped into a 4% alcoholic solution of a copolymer of 2-hydroxyethyl methacrylate (HEMA) and methacrylic acid (MAA) in a 1:1 molar ratio, followed by drying. The solution contained, in addition to the copolymer, 5 parts per hundred of partially methylated melamine-formaldehyde resin (as cross-linking agent) and 0.5 parts per hundred of paratoluene sulphonic acid (as catalyst).

The rubber was then vulcanised, after which the lubricity with respect to dry skin was subjectively evaluated on a scale of 1 to 5, in which:

1 means that the film is sticky

2 means that poor slip is obtained

3 means that moderate slip is obtained

4 means that quite good slip is obtained

5 means that excellent slip is obtained

(comparable to the use of a powdered surface)

The dry skin lubricity number was 5; the coating adhered satisfactorily to the rubber and no visible flaking was observed.

Examples 2 to 11

Example 1 was repeated, except that the copolymer was replaced by the polymers indicated in the following Table 1:

<div align="center">

Table 1

</div>

| Example No. | Polymer | Molar ratio of monomers | Dry skin lub-ricity number |
|---|---|---|---|
| 2 | HEMA/MAA | 2:1 | 5 |
| 3 | HEMA/MAA | 5:1 | 5 |
| 4 | HEMA/MAA | 10:1 | 5 |
| 5 | HEMA/EHA | 2.5:1 | 5 |
| 6 | HEMA/EHA | 4:1 | 5 |
| 7 | HEMA/EHA | 5:1 | 5 |
| 8 | HEMA/EHA | 10:1 | 5 |
| 9 | HEMA/MAA/EHA | 10:1:0.5 | 5 |
| 10 | HEMA/MAA/EHA | 5:1:1.2 | 5 |
| 11 | HEMA/MAA/BA | 10:1:0.5 | 5 |
| Comparative 1 | HEMA/AA | 2:1 | 4 |
| Comparative 2 | HEMA/AA | 1:1 | 4 |
| Comparative 3 | HEMA/MMA | 2:1 | 4 |
| Comparative 4 | HEMA/MMA | 1:1 | 4 |
| Comparative 5 | HEMA/BA | 5:1 | 4 |
| Comparative 6 | HEMA/BA | 2:1 | 3 |
| Comparative 7 | HEMA/MA | 2:1 | 4 |
| Comparative 8 | HEMA/IA | 2:1 | 4 |
| Comparative 9 | HEMA/EHA | 2:1 | 4 |
| Comparative 10 | HEMA/EHA | 1:1 | 4 |
| Comparative 11 | MMA/VPd | 1:1 | 3 |
| Comparative 12 | HEMA | – | 3-4 |
| Comparative 13 | HEA | – | 3-4 |
| Comparative 14 | VPd | – | 2 |
| Comparative 15 | HPMA | – | 3-4 |
| Comparative 16 | HEMA/HEA | 1:1 | 4 |
| Comparative 17 | HEMA/VPd | 1:1 | 3-4 |

Table 1 (Continued)

| Example No. | Polymer | Molar ratio of monomers | Dry skin lubricity number |
|---|---|---|---|
| Comparative 18 | HEMA/HPMA | 1:1 | 4 |
| Comparative 19 | HEA/HPMA | 1:1 | 4 |
| Comparative 20 | HEMA/VPy | 9:1 | 4-5 |

In the above Table, the abbreviations have the following meanings:

EHA      2-ethylhexyl acrylate
BA      butyl acrylate
AA      acrylic acid
MMA      methyl methacrylate
MA      methyl acrylate
IA      itaconic acid
VPd      N-vinyl pyrrolidone
HEA      hydroxyethyl acrylate
HPMA      hydroxypropyl methacrylate
VPy      vinyl pyridine (quaternised)

It will be seen that the dry skin lubricity for each of the Examples according to the invention was better than that obtained in any of the Comparative Examples (the only comparative sample approaching the lubricity of the samples according to the invention was that of Comparative Example 20, when a quaternised copolymer was used).

In each of the Examples according to the invention, the coating adhered satisfactorily with at most very slight flaking. This also applied to the Comparative Examples, except Comparative Example 14 (where the coating was washed off on wet-stripping).

The dry frictional force and the coefficient of friction for the glove of Example 6, the glove of Example 10 and for a conventional powdered glove are given in the following Table 2.

Table 2

| Glove | Dry frictional force | Coefficient of friction |
|---|---|---|
| Example 6 | 48.7 g | 0.20 |
| Example 10 | 53.1 g | 0.218 |
| Conventional powdered | 78.5 g | 0.323 |

The damp skin lubricity number was 2 for Examples 1 to 11 and Comparative Examples 2 to 10, 12, 13, and 15 to 20, and 1 for Comparative Examples 1, 11 and 14.

Examples 12 to 21

Samples prepared as in Example 10 were post-treated by dipping in solutions of various materials, as identified in the following Table 3.

5

Table 3

| Example No. | Material | Concentration of solution | Damp skin lubricity number |
|---|---|---|---|
| 12 | N-cetylpyridinium chloride (N-CPC) | 5% | 3 - 4 |
| 13 | - do - | 1% | 3 - 4 |
| 14 | sodium lauryl sulphate | 5% | 3 |
| 15 | sodium lauryl sulphate | 1.0% | 3 |
| 16 | - do - | 0.5% | 3 |
| 17 | - do - | 0.1% | 2 - 3 |
| 18 | N,N-dimethyl hexadecylamine | 1% | 3 - 4 |
| 19 | Ethylene oxide-polypropylene glycol condensate | 1% | 3 |
| 20 | Distearyl dimethyl ammonium chloride | 1% | 3 |
| 21 | Hexadecyl trimethyl ammonium chloride | 1% | 3 - 4 |

In each case, the dry slip was substantially unimpaired.

Examples 22 to 26

Example 12 was repeated, using solutions containing various proportions of N-CPC and also 0.3% medical grade polydimethyl siloxane, as indicated in the following Table 4.

Table 4

| Example No. | Percentage N-CPC | Damp skin lubricity number |
|---|---|---|
| 22 | 0.1 | 3 |
| 23 | 0.25 | 3 - 4 |
| 24 | 0.50 | 4 |
| 25 | 1.0 | 4 |
| 26 | 2.0 | 4 |

Similar results to those of Example 22 were obtained when the percentage of polydimethyl siloxane was 0.05%.

Example 27

A series of hand-shaped formers were dipped into a natural rubber latex to produce a thin rubber layer on each former. The rubber layer was leached in hot water and then primed by dipping in dilute sulphuric acid, rinsed, dipped into a caustic soda bath or pH 10.5 containing hydrogen peroxide in an amount sufficient to react with hydrogen sulphide formed in the priming stage. The rubber, still on the formers, was then dipped into a 4% ethanolic solution of a HEMA/MAA/EHA terpolymer with a monomer molar ratio of 5:1:1.2, the solution also containing 5 to 15 parts per hundred (based on the weight of polymer) of partially methylated melamine-formaldehyde resin available commercially as Cymel 373 (as cross-linking agent) and 0.5 to 1.5 parts per hundred (on the same basis) of para-toluene sulphonic acid (as catalyst).

The rubber was then vulcanised and the polymer simultaneously cured (the temperature being raised from 80 to 150°C over 25 minutes during vulcanisation), the resulting gloves being stripped from the formers.

The stripped gloves were washed with water and then tumbled in an aqueous solution containing 0.75% by weight of N-cetylpyridinium chloride, the solution also containing 0.05% by weight of emulsified silicone. The gloves were finally tumbled dry at 65°C for 75 minutes.

The resulting gloves had a dry skin lubricity number of 5 and a wet skin lubricity number of 4 on their polymer-coated surfaces (used as the insides of the gloves).

No allergenic or irritant reaction to the gloves was reported, even when the gloves were worn by surgeons with hypersensitive skin.

Example 28

Example 1 was repeated except that the copolymer was replaced by a copolymer having the following molar percentage: 80% HEMA, 10% MAA, 10% EHA. A dry skin lubricity number of 5 was obtained.

Example 29

A rubber-coated porcelain mandrel was dipped for several minutes in water at above 70°C, rinsed in running water, and dipped in 2% sulphuric acid at 40°C. The coated mandrel was then dipped for (neutralisation) in dilute caustic (pH 9-10) and then dipped in water wash tanks at 40°C. The coated mandrel was then coated with a 10% solution in ethanol of a terpolymer as used in Example 10, the solution containing 10% by weight of Cymel 370 (cross-linking agent) and 1% by weight of p-toluenesulphonic acid.

The coated mandrel was heated in an oven for 30 minutes with temperatures rising to 105°C.

The glove was stripped from the mandrel and immersed for 15 minutes in an aqueous dispersion of 0.05. of 35% Silicone medical grade emulsion DC365 (Dow Corning brand) containing 0.5% Cetylpyridinium chloride. After draining, the glove was heated and dried in an oven for 30 minutes at 70°C.

The outer surface of the glove was tack-free; the inner coated surface was hydrophilic, had a high degree of slip and was readily donned on a dry hand (a dry skin lubricity number of 5).

The moisture transmission properties at 25°C (100% RH) of the resulting glove and an otherwise similar, but uncoated (control), glove are set out in the following Table 5.

Table 5

| Sample | Rate of moisture transmission $gm/m^2/mm/24$ hrs.) |
|---|---|
| According to the invention Control | 7.86 4.22 |

Examples 30 to 37

Example 1 was repeated, except that the copolymer was replaced by the polymers indicated in the following Table 6.

Table 6

| Example No. | Molar percentage HEMA | Molar percentage MAA | Molar percentage EHA |
|---|---|---|---|
| 30 | 70 | 20 | 10 |
| 31 | 60 | 10 | 30 |
| 32 | 60 | 20 | 20 |
| 33 | 60 | 30 | 10 |
| 34 | 50 | 30 | 20 |
| 35 | 40 | 30 | 30 |
| 36 | 40 | 40 | 20 |
| 37 | 30 | 60 | 10 |

In each case, a dry skin lubricity number of 5 was obtained.

Examples 38 to 53

Example 12 was repeated, using solutions of various surfactants instead of N-CPC. The results are summarised in the following Table 7.

7

Example 54

Example 1 was repeated, except that the copolymer was replaced by a copolymer having the following molar percentage: 70% HEMA, 10% MAA, 10% EHA. A dry skin lubricity number of 5 was obtained.

Table 7

| Example No. | Surfactant | Concentration of solution | Damp skin lubricity number |
|---|---|---|---|
| 38 | Octadecyl trimethyl ammonium bromide | 1.0% | 3 |
| 39 | Cetyl pyridinium bromide | 1.0% | 3-4 |
| 40 | 2-oleyl-1-(ethylbetaoxy propionic acid) imidazoline | 1.0% | 3 |
| 41 | Lauryl pyridinium chloride | 1.0% | 3-4 |
| 42 | Oxyethyl alkyl ammonium phosphate | 1.0% | 3 |
| 43 | 10-18C alkyl dimethyl benzyl ammonium chloride | 1.0% | 3 |
| 44 | Polypropoxy quaternary ammonium acetate | 1.0% | 3 |
| 45 | 12-14C alkylbetaine | 1.0% | 3 |
| 46 | Coconut imidazoline betaine | 1.0% | 3 |
| 47 | An amine oxide ethoxylate (Empigen DY) | 1.0% | 3 |
| 48 | N-cetyl-N-ethyl morpholinium ethosulphate | 1.0% | 3 |
| 49 | Distearyl dimethylammonium chloride | 1.0% | 3 |
| 50 | Pluronic F38 (an ethylene oxide-polypropylene glycol of mol.wt. 5020) | 0.15% | 3 |
| 51 | Polyethylene oxide (mol.wt. 4000000) | 1.0% | 3 |
| 52 | Dodecylbenzyl-hydroxymethyl dimethyl chloride | 1.0% | 3 |
| 53 | Cetyl stearyl ethylene oxide condensate (20 ethylene oxide units) | 5% | 3 |

8

**Claims**

1. A process for making a flexible dipped rubber article having a lubricating layer formed from a hydrogel polymer bonded thereto so as to provide a skin-contacting surface of said article, characterised in that the lubricating layer is formed by applying a coating of a solution of a hydrophilic hydrogel-forming polymer to the dipped rubber article prior to the complete vulcanisation thereof, and heating the coated rubber article to cure the polymer and to complete vulcanisation of the rubber.

2. A process according to claim 1, in which the coating of the polymer solution is formed by dipping.

3. A process according to claim 1 or 2, in which the skin-contacting surface has surfactant material applied thereto so as to substantially improve the lubricity of the surface with respect to damp skin.

4. A process according to claim 1 or 2, which comprises the steps of:
   (a) forming the rubber article by dipping a suitably shaped former in rubber latex,
   (b) leaching the rubber article on the former in hot water,
   (c) priming the rubber surface of the article on the former by means of a dilute acid,
   (d) rinsing the primed surface in water or aqueous alkali,
   (e) dipping said rubber article, while still on the former, in a solution of the hydrophilic, hydrogel-forming polymer and a curing agent therefor,
   (f) heating the resulting coating so that the resulting hydrogel polymer is bonded to the rubber and so that the rubber is vulcanised and the polymer is simultaneously cured, and
   (g) stripping the resulting article from the former.

5. A process according to claim 4, which further comprises;
   (h) applying a solution of surfactant material containing silicone to said coating of hydrogel polymer, and
   (i) heating the resultant coating of surfactant material so as to fix the slip properties of the coating.

**Patentansprüche**

1. Verfahren zum Herstellen eines flexiblen getauchten Gummiartikels, der eine aus einem Hydrogelpolymer gebildete gleitendmachende Schicht daran gebunden hat, so daß sie eine hautberührende Oberfläche des Artikels ergibt, **dadurch gekennzeichnet,** daß die gleitendmachende Schicht gebildet wird durch Auftragen eines Überzugs einer Lösung eines hydrophilen Hydrogel-bildenden Polymers auf den getauchten Gummiartikel vor dessen vollständiger Vulkanisierung und Erwärmen des überzogenen Gummiartikels, um das Polymer zu härten und die Vulkanisierung des Gummis zu vervollständigen.

2. Verfahren nach Anspruch 1, in welchem der Überzug aus der Polymerlösung durch Tauchen gebildet wird.

3. Verfahren nach Anspruch 1 oder 2, in welchem die hautberührende Oberfläche oberflächenaktives Material darauf aufgetragen hat, so daß die Gleitfähigkeit der Oberfläche im Hinblick auf feuchte Haut wesentlich verbessert wird.

4. Verfahren nach Anspruch 1 oder 2, welches die Schritte umfaßt:
   a) Bilden des Gummiartikels durch Eintauchen eines in geeigneter Weise gestalteten Formkörpers in Gummilatex,
   b) Auslaugen des Gummiartikels auf dem Formkörper in heißem Wasser,
   c) Vorbehandeln der Gummioberfläche des Artikels auf dem Formkörper mittels einer verdünnten Säure,
   d) Spülen der vorbehandelten Oberfläche in Wasser oder in wäßrigem Alkali,
   e) Eintauchen des Gummiartikels, der sich noch auf dem Formkörper befindet, in eine Lösung des hydrophilen, Hydrogel-bildenden Polymers und eines Härtungsmittels dafür,
   f) Erwärmen des daraus hervorgehenden Überzugs, so daß das daraus hevorgehende Hydrogelpolymer an den Gummi gebunden ist und so daß der Gummi vulkanisiert und das Polymer gleichzeitig gehärtet wird,
   g) Abstreifen des daraus hervorgehenden Artikels von dem Formkörper.

**5.** Verfahren nach Anspruch 4, welches weiter umfaßt:

h) Auftragen einer Lösung aus oberflächenaktivem Material, welches Silicon enthält, auf den Überzug aus Hydrogelpolymer, und

i) Erwärmen des daraus hervorgehenden Überzugs aus oberflächenaktivem Material, um die Gleiteigenschaften des Überzugs zu fixieren.

**Revendications**

**1.** Procédé de fabrication d'un article de trempage en caoutchouc souple sur lequel est fixée une couche lubrifiante formée à partir d'un polymère formateur d'hydrogel de manière à former sur ledit article une surface de contact avec la peau, caractérisé en ce que la couche lubrifiante est formée par application à l'article de trempage en caoutchouc, avant sa vulcanisation complète, d'un revêtement d'une solution d'un polymère formateur d'hydrogel hydrophile et chauffage de l'article en caoutchouc revêtu pour durcir le polymère et pour achever la vulcanisation du caoutchouc.

**2.** Procédé selon la revendication 1, dans lequel le revêtement de solution de polymère est formé par trempage.

**3.** Procédé selon la revendication 1 ou 2, dans lequel un tensioactif est appliqué sur la surface de contact avec la peau de manière à améliorer sensiblement le caractère lubrifiant de la surface par rapport à la peau humide.

**4.** Procédé selon la revendication 1 ou 2 qui comprend les étapes consistant à :

a) former l'article en caoutchouc par trempage d'un gabarit de forme appropriée dans un latex de caoutchouc,

b) lavage dans l'eau chaude de l'article en caoutchouc sur le gabarit,

c) apprêtage de la surface en caoutchouc de l'article sur le gabarit à l'aide d'un acide dilué,

d) rinçage de la surface apprêtée dans l'eau ou dans un alcali aqueux,

e) trempage dudit article en caoutchouc, tandis qu'il est encore sur le gabarit, dans une solution de polymère formateur d'hydrogel hydrophile et d'un agent de durcissement pour celui-ci,

f) chauffage du revêtement résultant de manière que le polymère formateur d'hydrogel résultant soit fixé au caoutchouc, et de manière que le caoutchouc soit vulcanisé et que le polymère soit durci simultanément, et

g) séparation de l'article résultant d'avec le gabarit.

**5.** Procédé selon la revendication 4, qui comprend en outre :

h) l'application d'une solution d'un tensioactif contenant une silicone sur ledit revêtement de polymère formateur d'hydrogel, et

i) le chauffage du revêtement de tensioactif résultant de manière à fixer les propriétés de glissement du revêtement.

10